# EUROPÄISCHE PATENTANMELDUNG

(11) **EP 2 172 228 A1**
(43) Veröffentlichungstag der Anmeldung: **07.04.2010**
(21) Anmeldenummer: 09011581.7
(22) Anmeldetag: 10.09.2009
(51) Int. Cl.: A61L 9/00, A61L 9/015, A61L 9/16, B01D 53/32, B03C 3/017, F24F 3/16

(54) **Ozonresistentes Filterelement**

(30) Priorität: 02.10.2008 DE 102008049927
(71) Anmelder: Carl Freudenberg KG, 69469 Weinheim (DE)
(72) Erfinder: Kögel, Markus, 67354 Römerberg (DE); Veeser, Klaus, 69469 Weinheim (DE); Reinhardt, Heinz, 68535 Edingen (DE)

(57) **Zusammenfassung**

Eine Verwendung eines Filterelements in einer ozonhaltigen Atmosphäre mit einer Ozonkonzentration von mindestens 1 ppm, welches ein Fasern aufweisendes Filtermedium umfasst, wobei die Fasern aus Polyester und/ oder Polycarbonat bestehen, löst die Aufgabe, ein Filterelement anzugeben, welches in einer ozonhaltigen Atmosphäre eine dauerhafte Betriebstauglichkeit zeigt.

## Beschreibung

### Technisches Gebiet

Die Erfindung betrifft die Verwendung eines Filterelements in einer ozonhaltigen Atmosphäre mit einer Ozonkonzentration von mindestens 1 ppm.

### Stand der Technik

In Kraftfahrzeugen kommen häufig Klimaanlagen zum Einsatz, die einen Ozongenerator aufweisen. Der Ozongenerator dient der Erzeugung von Ozongas, welches Bakterien abtöten soll. Eine solche Klimaanlage ist beispielsweise in der DE 10 2004 030 998 A1 beschrieben.

Des Weiteren ist es aus dem Stand der Technik bekannt, Filterelemente in den Ansaugtrakt einer Klimaanlage einzusetzen, um die angesaugte Luft zu reinigen.

Die Filterelemente in Klimaanlagen stehen häufig in unmittelbarer Strömungsverbindung zu einem Verdampfer, in dessen Nähe der Ozongenerator positioniert ist. Durch den Ozongenerator wird eine ozonhaltige Atmosphäre erzeugt, welche die aus dem Stand der Technik bekannten hocheffizienten Filterelemente allerdings erheblich schädigt.

Die aus dem Stand der Technik bekannten Filterelemente zeigen in einer ozonhaltigen Atmosphäre mit einer Ozonkonzentration von mindestens 1 ppm Auflösungserscheinungen. Sowohl die Fasern des Filtermediums als auch eventuell verwendete Bindemittel werden durch das Ozon angegriffen. Hierbei könnte der Polymerisationsgrad der verwendeten Polymere erniedrigt werden. Beim Einsatz von so genannten Kombifiltern kommt es außerdem zu einer Zerstörung des Aktivkohleverbunds durch das Auflösen des Bindemittels.

### Darstellung der Erfindung

Der Erfindung liegt daher die Aufgabe zugrunde, ein Filterelement anzugeben, welches in einer ozonhaltigen Atmosphäre eine dauerhafte Betriebstauglichkeit zeigt.

Die vorliegende Erfindung löst die vorgenannte Aufgabe durch die Merkmale des Patentanspruchs 1

Danach wird ein Filterelement in einer ozonhaltigen Atmosphäre mit einer Ozonkonzentration von mindestens 1 ppm verwendet, welches ein Fasern aufweisendes Filtermedium umfasst, wobei die Fasern aus Polyester und/ oder Polycarbonat bestehen.

Erfindungsgemäß ist in einem ersten Schritt zunächst erkannt worden, dass das Ozon die Fasern und eventuell verwendete Bindemittel auflösen kann, und dadurch die aus dem Stand der Technik bekannten Filterelemente irreparabel schädigt. In einem zweiten Schritt ist sodann durch einen glücklichen Griff erkannt worden, dass Fasern aus Polyester und Fasern aus Polycarbonat in einer ozonhaltigen Atmosphäre überraschend hinreichend stabil bleiben. Schließlich ist erkannt worden, dass ein Filtermedium, welches Fasern aus Polyester und/ oder Fasern aus Polycarbonat aufweist, einer Ozonkonzentration von mindestens 1 ppm über eine sehr lange Zeit derart standhalten kann, dass nahezu keine Schädigung des Filterelements in seiner Gesamtheit auftritt. Dabei ist insbesondere überraschend, dass die Fasern trotz ihrer geringen Durchmesser und ihrer großen Angriffsfläche für das Ozon stabil bleiben und eine dauerhaft betriebstaugliche Filtereffizienz des Filterelements gewährleisten.

Folglich ist die eingangs genannte Aufgabe gelöst.

Das Filterelement könnte aus zwei Lagen aus Vliesstoff gefertigt sein, wobei eine erste Lage Fasern aus Polyester und eine zweite Lage Fasern aus Polycarbonat aufweist. Ein Filtermedium aus Polyester und Polycarbonat hat sich als besonders stabil in einer ozonhaltigen Atmosphäre erwiesen.

Vor diesem Hintergrund könnte die zweite Lage mit Fasern aus Polycarbonat zwischen zwei Lagen mit Fasern aus Polyester eingebettet sein. Hierdurch werden die Fasern aus Polycarbonat durch die Fasern aus Polyester dauerhaft gestützt. Ein Absetzverhalten der Fasern aus Polycarbonat wird bei dem dreilagigen Filtermedium weitgehend verhindert.

Mindestens eine der Lagen könnte elektrostatisch aufgeladen sein. Durch diese konkrete Ausgestaltung können Feinstaubpartikel abgeschieden werden, die kleiner als 0,5 µm sind. Der mechanische Filtereffekt des Filtermediums wird durch einen elektrischen Filtereffekt unterstützt.

Vor diesem Hintergrund könnten die Fasern aus Polyester einen mittleren Durchmesser im Bereich 10 bis 50 µm aufweisen. Fasern mit einem solchen mittleren Durchmesser haben sich als besonders stabil erwiesen, um eine Stützlage für Fasern aus Polycarbonat auszubilden.

Dabei könnten die Fasern aus Polycarbonat einen mittleren Durchmesser von höchstens 10 µm aufweisen. Die Verwendung von so genannten Mikrofasern und/ oder Nanofasem aus Polycarbonat erlaubt eine zuverlässige mechanische Abscheidung von Feinstaubpartikeln.

Das Filtermedium könnte mit einer Lage aus Aktivkohle oder anderen Adsorbentien verbunden sein. Durch diese konkrete Ausgestaltung ist das Filterelement als so genannter Kombifilter ausgestaltbar. Die Adsorbentien, insbesondere Aktivkohlepartikel oder Aktivkohlefasern, könnten durch ein Bindemittel gebunden sein und mit dem Filtermedium laminiert sein. Hierdurch ist eine Adsorption von Gasen, insbesondere von unangenehmen Gerüchen, möglich. Bei der Positionierung eines solchen Filterelements in der Klimaanlage eines Kraftfahrzeugs ist die Lage aus Aktivkohle vorzugsweise dem Verdampfer und demozongenerator zugewandt. Eine dem Verdampfer und dem Ozongenerator abgewandte Positionierung der Lage aus Aktivkohle ist ebenfalls denkbar. Die Lage aus Aktivkohle kann zwischen einem dreilagigen Filtermedium und einer Trägerlage aus Polyester aufgenommen sein.

Das Filterelement könnte ein Bindemittel auf Polyurethanbasis aufweisen. Das Bindemittel auf Polyurethanbasis kann die Aktivkohlepartikel oder Aktivkohlefasern auch in einer ozonhaltigen Atmosphäre dauerhaft zuverlässig binden. Überraschenderweise hat sich gezeigt, dass Polyurethan in einer ozonhaltigen Atmosphäre chemisch und mechanisch stabil bleibt.

Das Filterelement könnte einen Fraktionsabscheidegrad gemäß DIN 71460 Teil 1 von mindestens 60 % für Partikeldurchmesser von 0,2 bis 1 µm nach einer Verweildauer von 240 Stunden in einer Atmosphäre mit einer Ozonkonzentration von 100 ppm zeigen. Ein Filterelement mit einem solchen Fraktionsabscheidegrad zeigt auch nach längerer Betriebsdauer eine hohe Filtereffizienz in Bezug auf Feinstaubpartikel.

Das Filterelement könnte einen Fraktionsabscheidegrad gemäß DIN 71460 Teil 1 von mindestens 70 % für Partikeldurchmesser von 1 bis 3 µm nach einer Verweildauer von 240 Stunden in einer Atmosphäre mit einer Ozonkonzentration von 100 ppm zeigen. Ein solches Filterelement weist auch nach längerer Betriebsdauer eine zuverlässige Filtereffizienz für mittlere Partikeldurchmesser auf.

Das Filterelement könnte einen Fraktionsabscheidegrad gemäß DIN 71460 Teil 1 von mindestens 80 % für Partikeldurchmesser von 3 bis 10 µm nach einer Verweildauer von 240 Stunden in einer Atmosphäre mit einer Ozonkonzentration von 100 ppm zeigen. Ein solches Filterelement zeigt eine hohe Filtereffizienz auch nach längerer Betriebsdauer einer ozonhaltigen Atmosphäre für grobe Partikel.

Vor diesem Hintergrund könnte das Filterelement in einer ozonerzeugenden Klimaanlage eines Kraftfahrzeugs verwendet werden. Durch die Verwendung des hier beschriebenen Filterelements in der Klimaanlage eines Kraftfahrzeugs kann dem Kraftfahrzeuginnenraum dauerhaft eine nahezu keimfreie und von Feinstaubpartikeln gereinigte Luft zugeführt werden. Dies ist eine besonderer kombinatorischer Effekt, der sich durch die konkrete Verwendung ergibt.

Es gibt nun verschiedene Möglichkeiten, die Lehre der vorliegenden Erfindung auf vorteilhafte Weise auszugestalten und weiterzubilden. Dazu ist einerseits auf die nachgeordneten Ansprüche, andererseits auf die nachfolgende Erläuterung eines bevorzugten Ausführungsbeispiels der Erfindung anhand der Zeichnung zu verzeichnen.

In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert.

### Kurzbeschreibung der Zeichnung

In der Zeichnung zeigen
- Fig. 1: ein Diagramm, in dem der Fraktionsabscheidegrad eines Filterelements mit Fasern aus Polyester und Polycarbonat mit dem Fraktionsabscheidegrad eines Filterelements mit Fasern aus Polypropylen verglichen ist,
- Fig. 2: ein Diagramm, in dem der Druckverlust zweier Filterelemente bei verschiedenen Volumenströmen verglichen ist,
- Fig. 3: ein Diagramm, bei welchem der Einfluss der Dauer der Ozonbelastung auf den Fraktionsabscheidegrad dargestellt ist,
- Fig. 4: ein Diagramm, in dem der Einfluss einer alternierenden Ozonbelastung auf den Fraktionsabscheidegrad eines Filterelements dargestellt ist, und
- Fig. 5: ein Diagramm, in dem der Einfluss der Dauer der Ozonbelastung auf den Fraktionsabscheidegrad für Partikel mit einem Durchmesser zwischen 0,3 und 0,5 µm dargestellt ist.

### Ausführung der Erfindung

Das Filterelement zur Verwendung in einer ozonhaltigen Atmosphäre mit einer Ozonkonzentration von mindestens 1 ppm umfasst ein gefaltetes Filtermedium des Typs AF909 der Firma Freudenberg Vliesstoffe KG, 69469 Weinheim, DE. Bei diesem Filtermedium handelt es sich um ein dreilagiges Filtermedium, welches zwei Lagen mit Fasern aus Polyester aufweist und eine Lage mit Fasern aus Polycarbonat. Die Flächengewichte der Lagen mit Fasern aus Polyester betragen 30 und 130 g/m². Die Fasern aus Polyester weisen einen mittleren Durchmesser von 10 bis 50 µm auf. Die Lage mit Fasern aus Polycarbonat ist elektrostatisch aufgeladen und weist ein Flächengewicht von 2 g/m² auf. Die Fasern aus Polycarbonat weisen einen mittleren Durchmesser von höchstens 10 µm auf. Die Lage mit Fasern aus Polycarbonat wird sandwichartig von den Lagen mit Fasern aus Polyester eingeschlossen.

Ein solches Filterelement wurde im Hinblick auf seinen Fraktionsabscheidegrad und sein Anströmverhalten in einer ozonhaltigen Atmosphäre mit einem Filterelement verglichen, welches ein Filtermedium mit Fasern auf Polyolefinbasis aufweist. Bei den Fasern auf Polyolefinbasis handelte es sich um Fasern aus Polypropylen. Bei dem Filtermedium handelte es sich um ein Filtermedium des Typs AF 935 der Firma Freudenberg Vliesstoffe KG, 69469 Weinheim, DE. Dieses Filtermedium ist einlagig aus Fasern aus Polypropylen aufgebaut. Die Fasern weisen einen Durchmesser von ca. 25 bis 30 µm auf. Das Filtermedium weist ein Flächengewicht von 70 g/m² auf.

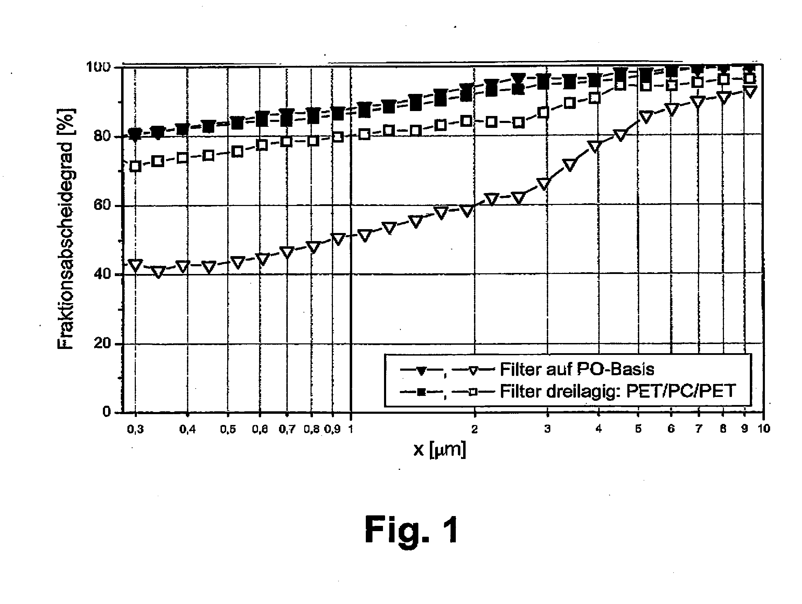

In Fig. 1 ist der Fraktionsabscheidegrad gemäß DIN 71460 Teil 1 in Abhängigkeit vom Durchmesser der zu filternden Partikel gezeigt. Der Fraktionsabscheidegrad wurde mit einem Teststaub A4 bei einer Belastung von 6 kg/min. gemessen. In dem Diagramm repräsentieren die geschlossenen Symbole die an Filterelementen im Neuzustand gemessenen Werte. Die offenen Symbole repräsentieren die Messwerte nach einer Verweildauer der Filterelemente von 240 Stunden in einer ozonhaltigen Atmosphäre mit einer Ozonkonzentration von 100 ppm. Die Dreiecksymbole repräsentieren ein Filterelement mit Fasern auf Polyolefinbasis. Die quadratischen Symbole repräsentieren das erfindungsgemäße Filterelement mit Fasern aus Polyester (PET) und Polycarbonat (PC).

Das Diagramm in Fig. 1 zeigt, dass das erfindungsgemäße Filterelement einen Fraktionsabscheidegrad gemäß DIN 71460 Teil 1 von mindestens 60 % für Partikeldurchmesser von 0,2 bis 1 µm nach einer Verweildauer von 240 Stunden in einer Atmosphäre mit einer Ozonkonzentration von 100 ppm aufweist. Das Filterelement weist des Weiteren einen Fraktionsabscheidegrad gemäß DIN 71460 Teil 1 von mindestens 70 % für Partikeldurchmesser von 1 bis 3 µm nach einer Verweildauer von 240 Stunden in einer Atmosphäre mit einer Ozonkonzentration von 100 ppm auf. Schließlich weist das Filterelement einen Fraktionsabscheidegrad gemäß DIN 71460 Teil 1 von mindestens 80 % für Partikeldurchmesser von 3 bis 10 µm nach einer Verweildauer von 240 Stunden in einer Atmosphäre mit einer Ozonkonzentration von 100 ppm auf

In Fig. 1 ist deutlich erkennbar, dass der Fraktionsabscheidegrad des Filterelements mit Fasern auf Polyolefinbasis nach der Verweildauer deutlich geringer ist als der des erfindungsgemäßen Filterelements. Bei dem erfindungsgemäßen Filterelement, welches ein dreilagiges Filtermedium des Typs AF90X verwendet, wird ein Fraktionsabscheidegrad von 75 % für Partikeldurchmesser zwischen 0,3 und 0,5 µm erreicht.

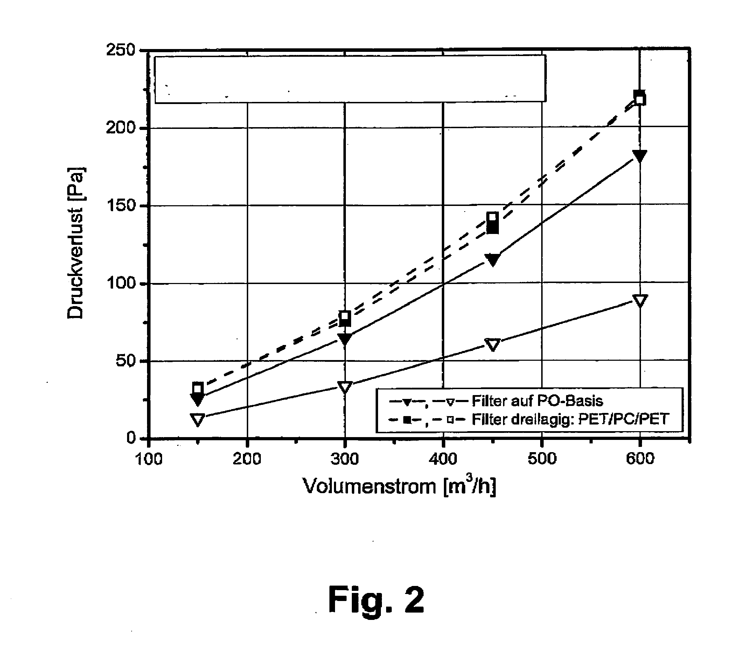

Fig. 2 zeigt ein Diagramm, in dem der Druckverlust zweier Filterelemente bei verschiedenen Volumenströme verglichen ist. In Fig. 2 werden dieselben Filterelemente verglichen wie in Fig. 1. In dem Diagramm repräsentieren die geschlossenen Symbole die an Filterelementen im Neuzustand gemessenen Werte. Die offenen Symbole repräsentieren die Messwerte nach einer Verweildauer der Filterelemente von 240 Stunden in einer ozonhaltigen Atmosphäre mit einer Ozonkonzentration von 100 ppm. Die Dreiecksymbole repräsentieren ein Filterelement mit Fasern auf Polyolefinbasis. Die quadratischen Symbole repräsentieren das erfindungsgemäße Filterelement mit Fasern aus Polyester (PET) und Polycarbonat (PC).

Das Diagramm gemäß Fig. 2 zeigt, dass das erfindungsgemäße Filterelement einen Druckverlust zwischen Anströmseite und Abströmseite von mindestens 200 PA bei einem Volumenstrom von 600 m³/h nach einer Verweildauer von 240 Stunden in einer Atmosphäre mit einer Ozonkonzentration von 100 ppm zeigt. Des Weiteren zeigt das erfindungsgemäße Filterelement einen Druckverlust zwischen Anströmseite und Abströmseite von mindestens 125 PA bei einem Volumeristrom von 450 m³/h nach einer Verweildauer von 240 Stunden in einer Atmosphäre mit einer Ozonkonzentration von 100 ppm. Das Filterelement zeichnet sich durch eine dauerhafte stabile Faserstruktur und dauerhaft stabile Porosität des Filtermediums aus.

In Fig. 2 ist deutlich erkennbar, dass das Filterelement mit Fasern auf Polyolefinbasis nach der Verweildauer in einer ozonhaltigen Atmosphäre deutlich geringere Druckverluste aufweist als das erfindungsgemäße Filterelement. Dies deutet darauf hin, dass die Fasern aus Polypropylen durch das Ozon angegriffen und zerstört werden. Die Druckverluste des erfindungsgemäßen Filterelements vor und nach der Verweildauer in einer ozonhaltigen Atmosphäre sind näherungsweise konstant. Dies belegt die mechanische und chemische Stabilität der Fasern aus Polyester und Polycarbonat gegenüber Ozon.

Bei einer Verwendung eines Filterelements in einer Klimaanlage eines Kraftfahrzeugs ist möglich, dieses nicht konstant mit Ozon zu beaufschlagen, sondern alternierdend, wie dies in DE 10 2004 030 998 A1 beschrieben ist.

Unter der Annahme, dass das Filterelement täglich etwa eine Stunde mit Ozon beaufschlagt wird, ergibt sich bei einer Fiitereinsatzzeit von zwei Jahren eine maximale Verweildauer in einer ozonhaltigen Atmosphäre von ca. 720 Stunden.

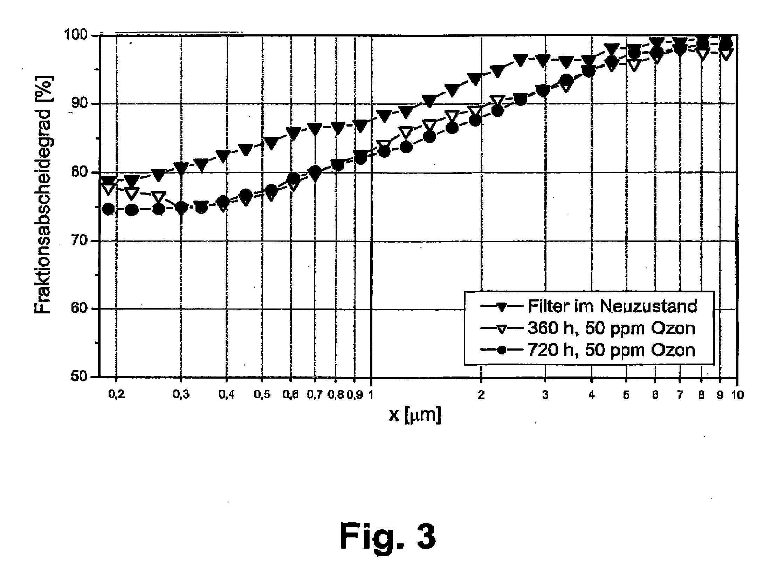

Fig. 3 zeigt den Einfluss der Verweildauer des erfindungsgemäßen Filterelements in einer ozonhaltigen Atmosphäre auf dessen Fraktionsabscheidegrad. Der Fraktionsabscheidegrad wurde analog zu Fig. 1 gemäß DIN 71460 Teil 1 mit Teststaub A4 und einer Beaufschlagung von 6 kg/min. gemessen. In Fig. 3 repräsentieren die geschlossenen Dreiecksymbole Messwerte, die an einem Filterelement im Neuzustand gemessen wurden. Die offenen Dreieckssymbole repräsentieren Messwerte, die nach einer Verweildauer von 360 Stunden in einer ozonhaltigen Atmosphäre mit einer Ozonkonzentration von 50 ppm gemessen wurden. Die geschlossenen Kreissymbole repräsentieren Messwerte, die nach einer Verweildauer von 720 Stunden in einer ozonhaltigen Atmosphäre mit einer Ozonkonzentration von 50 ppm gemessen wurden.

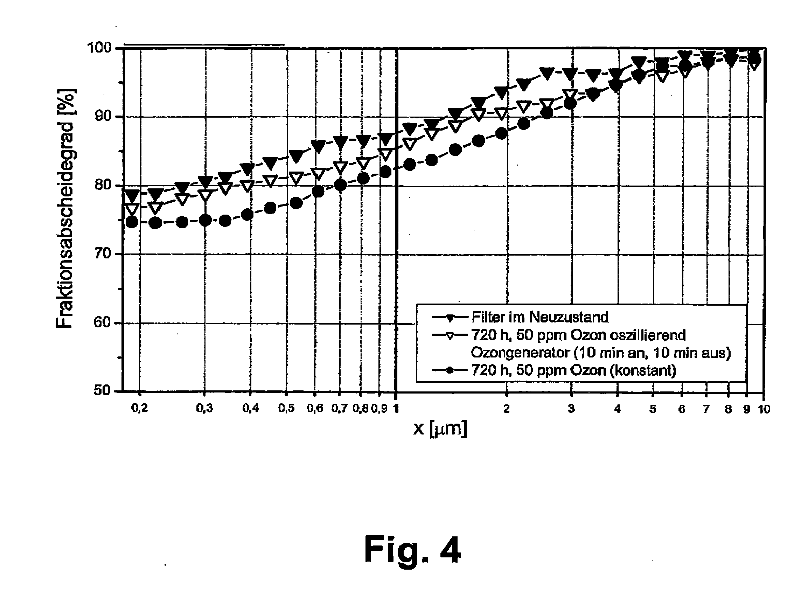

Fig. 4 zeigt den Einfluss einer alternierenden (oszillierenden) Ozonkonzentration auf das erfindungsgemäße Filterelement. Die Messungen wurden gemäß DIN 71460 Teil 1 durchgeführt. In Fig. 4 repräsentieren die geschlossenen Dreieckssymbole Messwerte, die an einem Filterelement im Neuzustand gemessen wurden Die offenen Dreieckssymbole repräsentieren Messwerte, die nach 720 Stunden Verweildauer in einer ozonhaltigen Atmosphäre mit einer Ozonkonzentration von 50 ppm bei alternierender Ozonbeaufschlagung gemessen wurden. Dabei wurde der Ozongenerator jeweils 10 min. betrieben und jeweils 10 min. ausgeschaltet. Die geschlossenen Kreissymbole repräsentieren Messwerte, die an einem Filterelement durchgeführt wurden, welches 720 Stunden in einer ozonhaltigen Atmosphäre mit einer Ozonkonzentration von 50 ppm konstant mit Ozon beaufschlagt wurden.

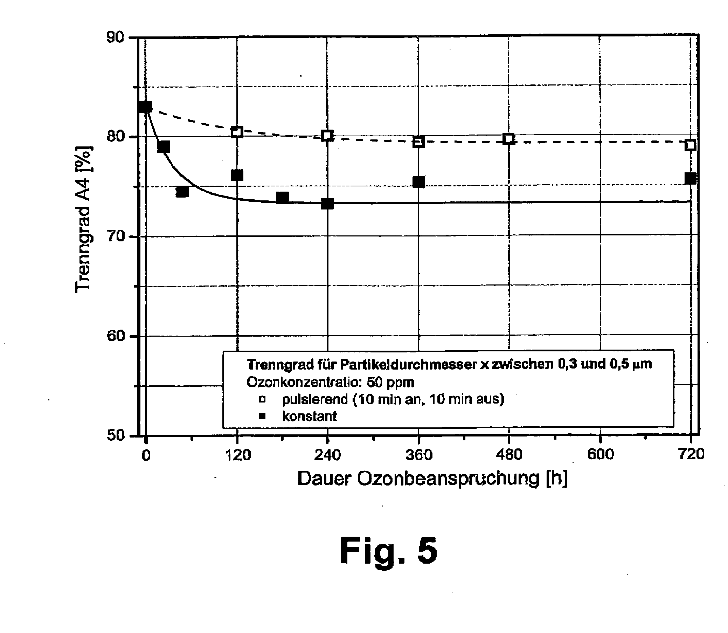

Fig. 5 zeigt ein Diagramm, in dem der Fraktionsabscheidegrad in Abhängigkeit von der Verweildauer zweier erfindungsgemäßer Filterelemente in ozonhaltiger Atmosphäre gezeigt ist. Der Fraktionsabscheidegrad (Trenngrad A4) bezieht sich konkret auf Partikel mit einem Partikeldurchmesser X zwischen 0,3 und 0,5 µm. In Fig. 5 repräsentieren die offenen Quadrate Messwerte, die an einem erfindungsgemäßen Filterelement bei alternierender Ozonbeanspruchung gemessen wurden. Die gefüllten Quadrate repräsentieren Messwerte, die bei konstanter Ozonbeanspruchung gemessen wurden.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

Abschließend sei ganz besonders hervorgehoben, dass das zuvor ausgewählte Ausführungsbeispiel lediglich zur Erörterung der erfindungsgemäßen Lehre dient, diese jedoch nicht auf dieses Ausführungsbeispiel einschränkt.

## Patentansprüche

1. Verwendung eines Filterelements in einer ozonhaltigen Atmosphäre mit einer Ozonkonzentration von mindestens 1 ppm, welches ein Fasern aufweisendes Filtermedium umfasst, wobei die Fasern aus Polyester und/oder Polycarbonat bestehen.

2. Verwendung nach Anspruch 1, **dadurch gekennzeichnet, dass** das Filtermedium aus zwei Lagen aus Vliesstoff gefertigt ist, wobei eine erste Lage Fasern aus Polyester und eine zweite Lage Fasern aus Polycarbonat aufweist.

3. Verwendung nach Anspruch 2, **dadurch gekennzeichnet, dass** die zweite Lage mit Fasern aus Polycarbonat zwischen zwei Lagen mit Fasern aus Polyester eingebettet ist.

4. Verwendung nach Anspruch 2 oder 3, **dadurch gekennzeichnet, dass** mindestens eine der Lagen elektrostatisch aufgeladen ist.

5. Verwendung nach einem der Ansprüche 2 bis 4, **dadurch gekennzeichnet, dass** die Fasern aus Polyester einen mittleren Durchmesser im Bereich 10 bis 50 µm aufweisen.

6. Verwendung nach einem der Ansprüche 2 bis 5, **dadurch gekennzeichnet, dass** die Fasern aus Polycarbonat einen mittleren Durchmesser von höchstens 10 µm aufweisen.

7. Verwendung nach einem der Ansprüche 1 bis 6, **dadurch gekennzeichnet, dass** das Filtermedium mit einer Lage aus Aktivkohle verbunden ist.

8. Verwendung nach einem der Ansprüche 1 bis 7, **dadurch gekennzeichnet, dass** das Filterelement ein Bindemittel auf Polyurethanbasis aufweist.

9. Verwendung nach einem der Ansprüche 1 bis 8, **dadurch gekennzeichnet, dass** das Filterelement einen Fraktionsabscheidegrad gemäß DIN 71460 Teil 1 von mindestens 60 % für Partikeldurchmesser von 0,2 bis 1 µm nach einer Verweildauer von 240 Stunden in einer Atmosphäre mit einer Ozonkonzentration von 100 ppm aufweist.

10. Verwendung nach einem der Ansprüche 1 bis 9, **dadurch gekennzeichnet, dass** das Filterelement einen Fraktionsabscheidegrad gemäß DIN 71460 Teil 1 von mindestens 70 % für Partikeldurchmesser von 1 bis 3 µm nach einer Verweildauer von 240 Stunden in einer Atmosphäre mit einer Ozonkonzentration von 100 ppm aufweist.

11. Verwendung nach einem der Ansprüche 1 bis 10, **dadurch gekennzeichnet, dass** das Filterelement einen Fraktionsabscheidegrad gemäß DIN 71460 Teil 1 von mindestens 80 % für Partikeldurchmesser von 3 bis 10 µm nach einer Verweildauer von 240 Stunden in einer Atmosphäre mit einer Ozonkonzentration von 100 ppm aufweist.

12. Verwendung nach einem der voranstehenden Ansprüche, **dadurch gekennzeichnet, dass** das Filterelement in einer ozonerzeugenden Klimaanlage eines Kraftfahrzeugs eingesetzt wird.
